# EUROPEAN PATENT SPECIFICATION

(11) **EP 4 054 482 B1**
(45) Date of publication and mention of the grant of the patent: **19.11.2025**
(21) Application number: 20811831.5
(22) Date of filing: 29.10.2020
(51) Int. Cl.: A61F 2/24, D03D 13/00

(54) **SKIRT ASSEMBLY FOR IMPLANTABLE PROSTHETIC VALVE**
SCHÜRZE FÜR IMPLANTIERBARE HERZKLAPPENPROTHESE
ENSEMBLE JUPE POUR VALVE PROTHÉTIQUE IMPLANTABLE

(30) Priority: 06.11.2019 US 201962931304 P
(43) Date of publication of application: 14.09.2022
(73) Proprietor: Edwards Lifesciences Corporation, Irvine, CA 92614 (US)
(72) Inventor: BUKIN, Michael, 30889 Caesarea (IL); LEVI, Tamir S., 30889 Caesarea (IL)
(74) Representative: Eisenführ Speiser
(86) International application number: PCT/US2020/057846
(87) International publication number: WO 2021/091754

(56) References cited:
- US-A1- 2012 165 929
- US-A1- 2016 220 359
- US-A1- 2016 317 305
- US-A1- 2019 046 314
- US-A1- 2019 053 897
- US-A1- 2019 192 296

## Description

### CROSS-REFERENCE TO RELATED APPLICATION

This application claims the benefit of U.S. Provisional Application No. 62/931,304, filed November 6, 2019.

### FIELD

The present disclosure concerns embodiments of a prosthetic valve for implantation into body ducts, such as native heart valve annuluses.

### BACKGROUND

The human heart can suffer from various valvular diseases. These valvular diseases can result in significant malfunctioning of the heart and ultimately require replacement of the native valve with an artificial valve. There are a number of known artificial valves and a number of known methods of implanting these artificial valves in humans.

Various surgical techniques may be used to replace or repair a diseased or damaged valve. Due to stenosis and other heart valve diseases, thousands of patients undergo surgery each year wherein the defective native heart valve is replaced by a prosthetic valve. Another less drastic method for treating defective valves is through repair or reconstruction, which is typically used on minimally calcified valves. The problem with surgical therapy is the significant risk it imposes on these chronically ill patients with high morbidity and mortality rates associated with surgical repair.

When the native valve is replaced, surgical implantation of the prosthetic valve typically requires an open-chest surgery during which the heart is stopped and patient placed on cardiopulmonary bypass (a so-called "heart-lung machine"). In one common surgical procedure, the diseased native valve leaflets are excised and a prosthetic valve is sutured to the surrounding tissue at the valve annulus. Because of the trauma associated with the procedure and the attendant duration of extracorporeal blood circulation, some patients do not survive the surgical procedure or die shortly thereafter. It is well known that the risk to the patient increases with the amount of time required on extracorporeal circulation. Due to these risks, a substantial number of patients with defective native valves are deemed inoperable because their condition is too frail to withstand the procedure. By some estimates, more than 50% of the subjects suffering from valve stenosis who are older than 80 years cannot be operated on for valve replacement.

Because of the drawbacks associated with conventional open-heart surgery, percutaneous and minimally-invasive surgical approaches are garnering intense attention. In one technique, a prosthetic valve is configured to be implanted in a much less invasive procedure by way of catheterization. For instance, U.S. Patent Nos. 5,411,522 and 6,730,118, describe collapsible transcatheter heart valves that can be percutaneously introduced in a compressed state on a catheter and expanded in the desired position by balloon inflation or by utilization of a self-expanding frame or stent.

Known prosthetic valves include a frame with a valvular structure (e.g., leaflets) mounted therein, an inner skirt secured to the inside of the frame, and optionally, an outer skirt secured to the exterior of the frame. The inner skirt can serve several functions. For example, the inner skirt can function as a seal member to prevent (or decrease) perivalvular leakage, to anchor the leaflets to the frame, and to protect the leaflets against damage caused by contact with the frame during crimping and during working cycles of the valve. The outer skirt can cooperate with the inner skirt to further reduce or avoid perivalvular leakage after implantation of the valve. The inner skirt desirably includes a tough, tear resistant material such as polyethylene terephthalate (PET), although various other synthetic or natural materials can be used.

For example, US 2019/0053897 A1 (US'897) discloses an implantable prosthetic valve with an annular frame having a plurality of frame members and a skirt assembly. The skirt assembly includes a laminate having a fabric layer sandwiched between a first covering member and a second covering member. At least one surface of the fabric layer can be exposed at one or more windows in the second covering member. The skirt assembly can be coupled to the annular frame by a suture extending through the fabric layer at the one or more windows and around at least one of the plurality of frame members.

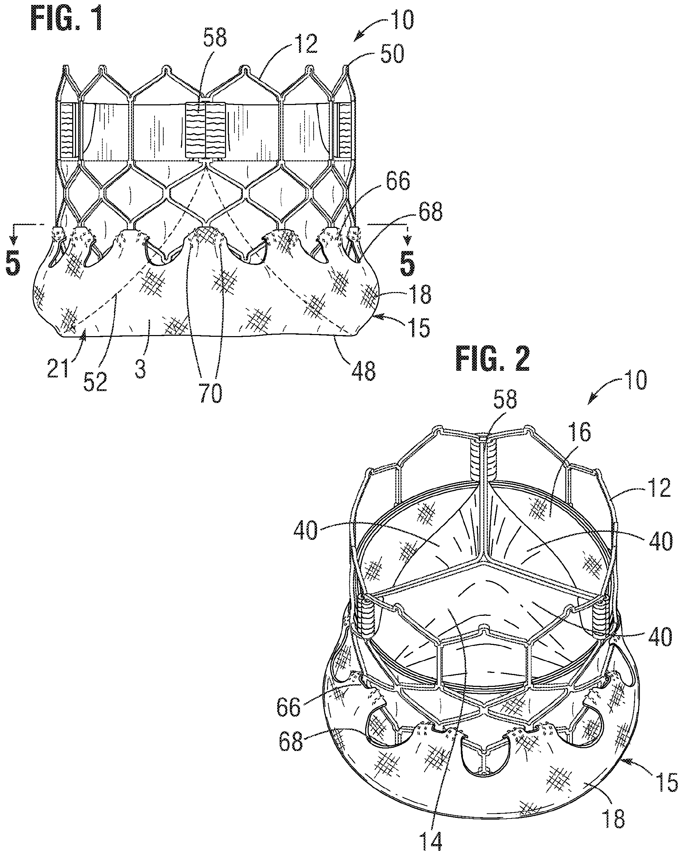

FIGS. 1 to 13 of US'897 are reproduced in the present application as FIGS. 1 to 13.

Another exemplary prosthetic heart valve is disclosed in US 2016/0220359 A1 (US'359). As shown in Fig. 7A of US'359, said document discloses a tubular seal which can include an inflow end section and an outflow end section. The inflow end section can include a fabric embedded within elastomeric material and the outflow end section can include a plurality of grommets. The fabric of inflow end section can be a woven material (cf. [0080] of US'359). In some cases, woven fabric can be arranged to have the warp and the waft extend in directions oblique to the axis of the tubular seal. In some cases, both the warp and the weft can extend at an angle between 30 degrees and 60 degrees with the axis of the tubular seal (cf. [0084] of US'359).

The inner and outer skirts are frequently secured to the frame by suturing or stitching the fabric of the respective skirts to the frame. Suturing of the inner skirt to the frame can expose the leaflets to the sutures. During working cycles of the valve, repetitive contact between the leaflets and the exposed sutures as well as contact between the leaflets and the fabric material of the skirt can cause abrasion of the leaflets. Accordingly, improvements to skirts for prosthetic valves are desirable.

### SUMMARY

The present disclosure is directed toward methods and apparatuses relating to prosthetic valves, such as prosthetic heart valves.

The present invention relates to an implantable prosthetic valve as defined in independent claim 1. The implantable prosthetic valve includes an annular frame that is radially expandable from a radially compressed state to a radially expanded state. The frame has an inflow end, an outflow end, and a longitudinal axis extending from the inflow end to the outflow end. The prosthetic valve also includes a plurality of leaflets positioned to regulate a flow of blood from the inflow end to the outflow end of the frame, and a skirt assembly. The skirt assembly includes a laminate having a textile layer sandwiched between a first encapsulation layer and a second encapsulation layer. The first and second encapsulation layers are made of an elastomer and the textile layer comprises a first set of yarns and a second set of yarns interwoven with the first set of yarns, wherein the first and second sets of yarns are non-perpendicular and non-parallel to the longitudinal axis. Moreover, the textile layer has a weave density of less than 150 ppi, i.e. 150 picks per inch, wherein one inch equals 2,54 centimetres.

Preferred configurations of the claimed implantable prosthetic valve are defined in dependent claims 2 to 14.

In some embodiments, and as defined in dependent claim 2, the textile layer can have a weave density of 50 ppi or less.

In some embodiment, and as also defined in dependent claim 2, the textile layer can have a weave density from about 30 to about 50 ppi.

In some embodiments, and as defined in dependent claim 3, the skirt assembly can include an outer skirt that is mounted to an outer surface of the frame.

In some embodiments, and as defined in dependent claim 4, the skirt assembly can have an inner skirt that is mounted to an inner surface of the frame.

In some embodiments, and as defined in dependent claim 5, the textile layer can be a braided layer.

In some embodiments, and as defined in dependent claim 6, the yarns of the first and second sets of yarns can be oriented at angles in the range of 20 to 70 degrees relative to the longitudinal axis of the frame.

In some embodiments, and as also defined in dependent claim 6, the yarns of the first and second sets of yarns can be oriented at angles in the range of 30 to 60 degrees relative to the longitudinal axis of the frame.

In some embodiments, and as also defined in dependent claim 6, the yarns of the first and second sets of yarns can be oriented at angles in the range of 40 to 50 degrees relative to the longitudinal axis of the frame.

In some embodiments, and as also defined in dependent claim 6, the yarns of the first and second sets of yarns can be oriented at 45-degree angles relative to the longitudinal axis of the frame.

In some embodiments, and as defined in dependent claim 7, the yarns of the first and second sets of yarns can have about 10 to about 50 filaments per yarn.

In some embodiments, and as also defined in dependent claim 7, the yarns of the first and second sets of yarns can have about 20 filaments per yarn.

In some embodiments, and as defined in dependent claim 8, the filaments of the yarns can have a thickness ranging from about 8 microns to about 16 microns.

In some embodiments, and as also defined in dependent claim 8, the filaments of the yarns can have a thickness about 10 microns.

In some embodiments, and as defined in dependent claim 9, at least some of the yarns of the first and second sets of yarns can be texturized such that they can retain a twisted or coiled state when the prosthetic valve is in the radially expanded state and are straightened to an untwisted or uncoiled state when the prosthetic valve is in the radially compressed state.

In some embodiments, and as defined in dependent claim 10, the textile layer can include leno woven yarns.

In some embodiments, and as defined in dependent claim 11, the yarns of the first and second sets of yarns can include a first type of yarns and a second type of yarns. The first type of yarns can be non-elastic or less elastic than the second type of yarns.

In some embodiments, and as defined in dependent claim 12, the textile layer can include a third set of yarns extending axially and braided together with the first and second sets of yarns to form a triaxial braid.

In some embodiments, and as defined in dependent claim 13, the third set of yarns can be made of an elastomer that is configured to stretch axially when the prosthetic valve is radially compressed from the radially expanded state to the radially compressed state.

In some embodiments, and as also defined in dependent claim 13, the third set of yarns can be texturized such that they can retain a twisted or coiled state when the prosthetic valve is in the radially expanded state and straightened to an untwisted or uncoiled state when the prosthetic valve is in the radially compressed state.

In some embodiments, and as defined in dependent claim 14, when the prosthetic valve is in the radially compressed state, the skirt assembly can be elongated axially up to at least 40% of its initial length when the prosthetic valve is in the radially expanded state.

In another representative example of the present disclosure, an implantable prosthetic valve can include an annular frame that is radially expandable from a radially compressed state to a radially expanded state. The frame can have an inflow end and an outflow end. The prosthetic valve can also include a plurality of leaflets positioned to regulate a flow of blood from the inflow end to the outflow end of the frame and an outer skirt mounted to an outer surface of the frame. The outer skirt can include a laminate having a textile layer sandwiched between a first encapsulation layer and a second encapsulation layer. The first and second encapsulation layers can be made of an elastomer and the textile layer can include a first set of yarns and a second set of yarns interwoven with the first set of yarns. The outer skirt can have a first axial length when the prosthetic valve is in the radially expanded state and a second axial length when the prosthetic valve is in the radially compressed state. The second axial length can be greater than 40% of the first axial length.

In some examples, the outer skirt can be configured to form a snug fit with the frame such that the outer skirt lies against the outer surface of the frame when the prosthetic valve is in the radially expanded state.

In some examples, the first and second sets of yarns can be respectively parallel to corresponding struts of the frame to which the outer skirt is connected when the prosthetic valve is in the radially expanded state.

In some examples, the first and second sets of yarns can be non-perpendicular and non-parallel to a longitudinal axis of the frame extending from the inflow end to the outflow end.

In some examples, the yarns of the first and second sets of yarns can be oriented at angles in the range of 20 to 70 degrees relative to the longitudinal axis of the frame.

In some examples, the yarns of the first and second sets of yarns can be oriented at angles in the range of 30 to 60 degrees relative to the longitudinal axis of the frame.

In some examples, the yarns of the first and second sets of yarns can be oriented at angles in the range of 40 to 50 degrees relative to the longitudinal axis of the frame.

In some examples, the yarns of the first and second sets of yarns can be oriented at 45-degree angles relative to the longitudinal axis of the frame.

In some examples, the textile layer can have a weave density of less than 150 ppi.

In some examples, the textile layer can have a weave density of 50 ppi or less.

In some examples, the textile layer can have a weave density from about 30 to about 50 ppi.

In some examples, at least some of the yarns of the first and second sets of yarns can be texturized such that they can retain a twisted or coiled state when the prosthetic valve is in the radially expanded state and are straightened to an untwisted or uncoiled state when the prosthetic valve is in the radially compressed state.

In some examples, the yarns of the first and second sets of yarns can include a first type of yarns and a second type of yarns. The first type of yarns can be non-elastic or less elastic than the second type of yarns.

In some examples, the textile layer can include a third set of yarns extending axially and braided together with the first and second sets of yarns to form a triaxial braid.

In some examples, the third set of yarns can be made of an elastomer that is configured to stretch axially when the prosthetic valve is radially compressed from the radially expanded state to the radially compressed state.

In some examples, the third set of yarns can be texturized such that they can retain a twisted or coiled state when the prosthetic valve is in the radially expanded state and straightened to an untwisted or uncoiled state when the prosthetic valve is in the radially compressed state.

Certain examples of the disclosure also concerns a method of assembling a prosthetic valve. The method can include mounting a skirt assembly to an annular frame, and attaching a plurality of leaflets to the annular frame. The frame can be radially expandable from a radially compressed state to a radially expanded state. The leaflets can be configured to regulate a flow of blood from an inflow end to an outflow end of the frame. The skirt assembly can include a laminate having a textile layer sandwiched between a first encapsulation layer and a second encapsulation layer. The first and second encapsulation layers can be made of an elastomer and the textile layer can include a first set of yarns and a second set of yarns interwoven with the first set of yarns. The outer skirt can have a first axial length when the prosthetic valve is in the radially expanded state and a second axial length when the prosthetic valve is in the radially compressed state. The second axial length can be up to at least 40% of the first axial length.

In some examples, the skirt assembly can include an inner skirt. The act of attaching the plurality of leaflets to the annular frame can include mounting the inner skirt to an inner surface of the frame and suturing the plurality of leaflets to the inner skirt.

In some examples, the skirt assembly can include an outer skirt. The act of attaching the skirt assembly to the annular frame can include placing the outer skirt around an outer surface of the frame and suturing the outer skirt to selected struts of the frame.

In some examples, the method can further include forming the laminate by forming the first encapsulation layer by electrospinning, placing the textile layer on the first encapsulation layer, and forming the second encapsulation layer on the textile layer by electrospinning.

In some examples, the method can further include forming the laminate by dipping the textile layer in a liquefied polymeric material, and then allowing the liquefied polymeric material to cure.

In some examples, the method can further include preparing the textile layer such that the first and second sets of yarns are non-perpendicular and non-parallel to a longitudinal axis of the frame extending from the inflow end to the outflow end.

In some examples, preparing the textile layer can include weaving the first and second set of yarns at selected angles relative to upper and lower edges of a fabric.

In some examples, preparing the textile layer can include cutting diagonally from a fabric whose woven yarns extend perpendicular to edges of the fabric.

In some examples, the yarns of the first and second sets of yarns can be oriented at angles in the range of 20 to 70 degrees relative to the longitudinal axis of the frame.

In some examples, the yarns of the first and second sets of yarns can be oriented at angles in the range of 30 to 60 degrees relative to the longitudinal axis of the frame.

In some examples, the yarns of the first and second sets of yarns can be oriented at angles in the range of 40 to 50 degrees relative to the longitudinal axis of the frame.

In some examples, the yarns of the first and second sets of yarns can be oriented at 45-degree angles relative to the longitudinal axis of the frame.

In some examples, the textile layer can have a weave density of less than 150 ppi.

In some examples, the textile layer can have a weave density of 50 ppi or less.

In some examples, the textile layer can have a weave density from about 30 to about 50 ppi.

In some examples, the textile layer can include a third set of yarns extending axially and braided together with the first and second sets of yarns to form a triaxial braid.

In some examples, the third set of yarns can be made of an elastomer that is configured to stretch axially when the prosthetic valve changes from the radially expanded state to the radially compressed state.

In some examples, the third set of yarns can be texturized such that they can retain a twisted or coiled state when the prosthetic valve is in the radially expanded state and straightened to an untwisted or uncoiled state when the prosthetic valve is in the radially compressed state.

The present invention further concerns a method of assembling a prosthetic valve as defined in independent claim 15. The method includes the steps of mounting a plurality of leaflets to an annular frame that is radially expandable from a radially compressed state to a radially expanded state, and mounting a skirt assembly to the annular frame. The frame has an inflow end, an outflow end, and a longitudinal axis extending from the inflow end to the outflow end. The plurality of leaflets are configured to regulate a flow of blood from the inflow end to the outflow end of the frame. The skirt assembly includes a laminate having a textile layer sandwiched between a first encapsulation layer and a second encapsulation layer. The first and second encapsulation layers are made of an elastomer and the textile layer includes a first set of yarns and a second set of yarns interwoven with the first set of yarns. The first and second sets of yarns are non-perpendicular and non-parallel to the longitudinal axis. The textile layer has a weave density of less than 150 ppi.

In some embodiments, the textile layer can have a weave density of 50 ppi or less.

In some embodiments, the textile layer can have a weave density from about 30 to about 50 ppi.

In some embodiments, the skirt assembly can include an outer skirt that is mounted to an outer surface of the frame.

In some embodiments, the skirt assembly can include an inner skirt that is mounted to an inner surface of the frame.

In some embodiments, the textile layer can be a braided layer.

In some embodiments, the textile layer can be a woven layer.

In some embodiments, the textile layer can be a knitted layer.

In some embodiments, the yarns of the first and second sets of yarns can be oriented at angles in the range of 20 to 70 degrees relative to the longitudinal axis of the frame.

In some embodiments, the yarns of the first and second sets of yarns can be oriented at angles in the range of 30 to 60 degrees relative to the longitudinal axis of the frame.

In some embodiments, the yarns of the first and second sets of yarns can be oriented at angles in the range of 40 to 50 degrees relative to the longitudinal axis of the frame.

In some embodiments, the yarns of the first and second sets of yarns can be oriented at 45-degree angles relative to the longitudinal axis of the frame.

In some embodiments, the yarns of the first and second sets of yarns can have about 10 to about 50 filaments per yarn.

In some embodiments, the yarns of the first and second sets of yarns can have about 20 filaments per yarn.

In some embodiments, the filaments of the yarns can have a thickness ranging from about 8 microns to about 16 microns.

In some embodiments, the filaments of the yarns can have a thickness about 10 microns.

In some embodiments, at least some of the yarns of the first and second sets of yarns can be texturized such that they can retain a twisted or coiled state when the prosthetic valve is in the radially expanded state and are straightened to an untwisted or uncoiled state when the prosthetic valve is in the radially compressed state.

In some embodiments, the textile layer can include leno woven yarns.

In some embodiments, the yarns of the first and second sets of yarns can include a first type of yarns and a second type of yarns. The first type of yarns can be non-elastic or less elastic than the second type of yarns.

In some embodiments, the textile layer can include a third set of yarns extending axially and braided together with the first and second sets of yarns to form a triaxial braid.

In some embodiments, the third set of yarns can be made of an elastomer that is configured to stretch axially when the prosthetic valve is radially compressed from the radially expanded state to the radially compressed state.

In some embodiments, the third set of yarns can be texturized such that they can retain a twisted or coiled state when the prosthetic valve is in the radially expanded state and straightened to an untwisted or uncoiled state when the prosthetic valve is in the radially compressed state.

In some embodiments, when the prosthetic valve is in the radially compressed state, the skirt assembly can be elongated axially up to at least 40% of its initial length when the prosthetic valve is in the radially expanded state.

### BRIEF DESCRIPTION OF THE DRAWINGS

FIG. 1 shows a side elevation view of an exemplary embodiment of an implantable prosthetic valve.
FIG. 2 shows a top perspective view of the prosthetic valve of FIG. 1.
FIG. 3 shows an exemplary frame of the prosthetic valve of FIG. 1.
FIG. 4 shows is a flattened view of the frame shown in FIG. 3.
FIG. 5 shows is a cross-sectional view of the prosthetic valve of FIG. 1 taken along line 5-5 of FIG. 1.
FIG. 6 shows the formation of a first covering member on a mandrel according to an exemplary process of making the inner skirt of the prosthetic valve of FIG. 1.
FIG. 7 shows the placement of a fabric layer over the first covering member shown in FIG. 6 according to the exemplary process of making the inner skirt.
FIG. 8 shows the placement of a plurality of masks over the fabric layer shown in FIG. 7 according to the exemplary process of making the inner skirt.
FIG. 9 shows the formation of a second covering member over the fabric layer having masks shown in FIG. 8 according to the exemplary process of making the inner skirt.
FIG. 10 shows the removal of the masks after forming the second covering member shown in FIG. 9 according to the exemplary process of making the inner skirt.
FIG. 11 shows a cross-sectional view of a portion of an inner skirt having a window on one side of the inner skirt exposing the underlying woven fabric.
FIG. 12 shows threading a suture through the woven fabric at the window shown in FIG. 11.
FIG. 13 shows suturing the inner skirt shown in FIG. 11 to an adjacent strut of a prosthetic valve's frame.
FIG. 14 is a perspective view of a prosthetic valve, according to another embodiment.
FIG. 15 is a cross-sectional view of the prosthetic valve of FIG. 14 taken along line 15-15 of FIG. 14.
FIGS. 16A and 16B show portions of two interwoven yarns of a skirt when the skirt is in a relaxed state (corresponding to a radially expanded state of a prosthetic valve) and an axially stretched state (corresponding to a radially compressed state of the prosthetic valve), respectively.
FIG. 17 shows a section of a braided layer for the outer skirt of the prosthetic valve of FIGS. 14-15, according to one embodiment.

### DETAILED DESCRIPTION

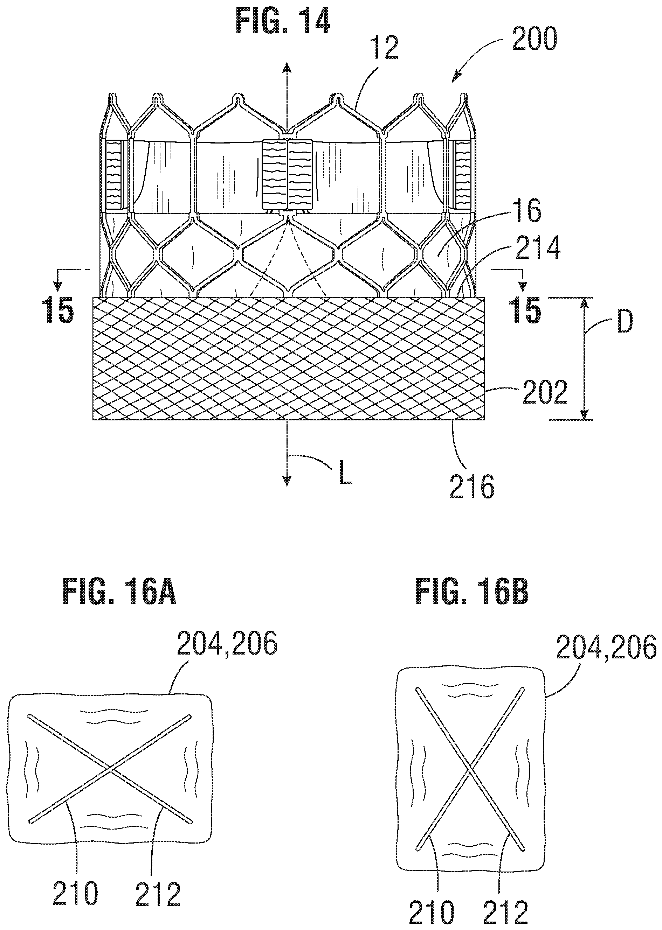

Specifics of the claimed invention and/or preferred embodiments of the claimed invention are described hereafter in particular in conjunction with FIGS. 14-17. Also described herein are related examples and arrangements useful for understanding the claimed invention.

FIGS. 1-2 show two different views of a prosthetic valve 10, according to one embodiment. The illustrated valve is adapted to be implanted in the native aortic annulus, although in other embodiments it can be adapted to be implanted in the other native annuluses of the heart. The valve 10 can have several main components: a stent, or frame, 12, a valvular structure 14, and a skirt assembly 15. The skirt assembly 15 can include an inner skirt 16, and optionally, an outer skirt 18.

The valvular structure 14 (or leaflet structure) can include three leaflets 40 (although a greater or fewer number of leaflets can be used), collectively forming the leaflet structure, which can be arranged to collapse in a tricuspid arrangement. The valvular structure 14 is configured to permit blood to flow through the prosthetic valve 10 in a direction from an inlet end 48 of the prosthetic valve to an outlet end 50 of the prosthetic valve and to block the flow of blood through the prosthetic valve in a direction from the outlet end 50 to the inlet end 48.

Each leaflet 40 desirably has a curved, generally U-shaped inlet or cusp edge 52. In this manner, the inlet edge of the valvular structure 14 has an undulating, curved scalloped shape. By forming the leaflets with this scalloped geometry, stresses on the leaflets can be reduced, which in turn improves durability of the valve. Moreover, by virtue of the scalloped shape, folds and ripples at the belly of each leaflet (the central region of each leaflet), which can cause early calcification in those areas, can be eliminated or at least minimized. The scalloped geometry also reduces the amount of tissue material used to form leaflet structure, thereby allowing a smaller, more even crimped profile at the inflow end of the valve. The leaflets 40 can be formed of pericardial tissue (e.g., bovine pericardial tissue), biocompatible synthetic materials, or various other suitable natural or synthetic materials as known in the art and described in U.S. Patent No. 6,730,118.

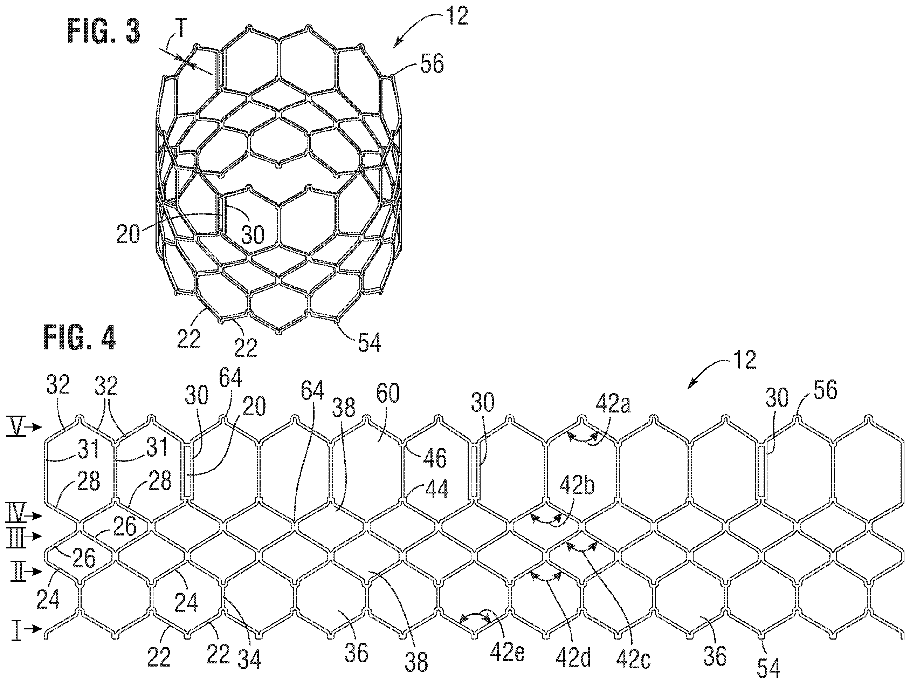

The bare frame 12 is shown in FIG. 3. In the depicted embodiment, the frame 12 has an annular shape defining an inlet end 54 and an outlet end 56 and includes a plurality of struts (or frame members). The frame 12 can be formed with a plurality of circumferentially spaced slots, or commissure windows, 20 (three in the illustrated embodiment) that are adapted to mount the commissures 58 of the valvular structure 14 to the frame, as described more fully in U.S. Patent Publication No. 2012/0123529.

The frame 12 can be made of any of various suitable plastically-expandable materials (e.g., stainless steel, etc.) or self-expanding materials (e.g., Nitinol) as known in the art. When constructed of a plastically-expandable material, the frame 12 (and thus the valve 10) can be crimped to a radially compressed state on a delivery catheter and then expanded inside a patient by an inflatable balloon or another suitable expansion mechanism. When constructed of a self-expandable material, the frame 12 (and thus the valve 10) can be crimped to a radially compressed state and restrained in the compressed state by insertion into a sheath or equivalent mechanism of a delivery catheter. Once inside the body, the valve can be advanced from the delivery sheath, which allows the valve to expand to its functional size.

Suitable plastically-expandable materials that can be used to form the frame 12 include, without limitation, stainless steel, a nickel based alloy (e.g., a cobalt-chromium or a nickel-cobalt-chromium alloy), polymers, or combinations thereof. In particular embodiments, frame 12 is made of a nickel-cobalt-chromium-molybdenum alloy, such as MP35N^{™} (tradename of SPS Technologies), which is equivalent to UNS R30035 (covered by ASTM F562-02). MP35N^{™}/UNS R30035 comprises 35% nickel, 35% cobalt, 20% chromium, and 10% molybdenum, by weight. It has been found that the use of MP35N to form frame 12 provides superior structural results over stainless steel. In particular, when MP35N is used as the frame material, less material is needed to achieve the same or better performance in radial and crush force resistance, fatigue resistances, and corrosion resistance. Moreover, since less material is required, the crimped profile of the frame can be reduced, thereby providing a lower profile valve assembly for percutaneous delivery to the treatment location in the body.

Referring to FIGS. 3 and 4, the frame 12 in the illustrated embodiment includes a first, lower row I of angled struts 22 arranged end-to-end and extending circumferentially at the inflow end of the frame; a second row II of circumferentially extending, angled struts 24; a third row III of circumferentially extending, angled struts 26; a fourth row IV of circumferentially extending, angled struts 28; and a fifth row V of circumferentially extending, angled struts 32 at the outflow end 56 of the frame. A plurality of substantially straight axially extending struts 34 can be used to interconnect the struts 22 of the first row I with the struts 24 of the second row II. The fifth row V of angled struts 32 are connected to the fourth row IV of angled struts 28 by a plurality of axially extending window frame portions 30 (which define the commissure windows 20) and a plurality of axially extending struts 31. Each axial strut 31 and each frame portion 30 extends from a location defined by the convergence of the lower ends of two angled struts 32 to another location defined by the convergence of the upper ends of two angled struts 28.

Each commissure window frame portion 30 mounts a respective commissure 58 of the leaflet structure 14. As can be seen, each frame portion 30 is secured at its upper and lower ends to the adjacent rows of struts to provide a robust configuration that enhances fatigue resistance under cyclic loading of the valve compared to known cantilevered struts for supporting the commissures of the leaflet structure. This configuration enables a reduction in the frame wall thickness to achieve a smaller crimped diameter of the valve. In particular embodiments, the thickness T of the frame 12 (FIG. 3) measured between the inner diameter and outer diameter is about 0.48 mm or less.

The struts and frame portions of the frame collectively define a plurality of open cells of the frame. At the inflow end of the frame 12, struts 22, struts 24, and struts 34 define a lower row of cells defining openings 36. The second, third, and fourth rows of struts 24, 26, and 28 define two intermediate rows of cells defining openings 38. The fourth and fifth rows of struts 28 and 32, along with frame portions 30 and struts 31, define an upper row of cells defining openings 60. The openings 60 are relatively large and are sized to allow portions of the leaflet structure 14 to protrude, or bulge, into and/or through the openings 60 when the frame 12 is crimped in order to minimize the crimping profile.

As shown in FIG. 4, the lower end of the strut 31 is connected to two struts 28 at a node or junction 44, and the upper end of the strut 31 is connected to two struts 32 at a node or junction 46. The strut 31 can have a thickness that is less than the thicknesses of the junctions 44, 46. The junctions 44, 46, along with junctions 64, each of which links two adjacent struts 32, prevent full closure of openings 60 when the frame 12 is in a crimped state. Thus, the geometry of the struts 31, and junctions 44, 46 and 64 assists in creating enough space in openings 60 in the crimped state to allow portions of the leaflets to protrude (i.e., bulge) outwardly through openings. This allows the valve to be crimped to a relatively smaller diameter than if all of the leaflet material is constrained within the crimped frame.

The frame 12 is configured to prevent or at least minimize possible over-expansion of the valve at a predetermined balloon pressure, especially at the outflow end portion of the frame, which supports the leaflet structure 14. In one aspect, the frame is configured to have relatively larger angles 42a, 42b, 42c, 42d, 42e between struts. The larger the angle, the greater the force required to open (expand) the frame. As such, the angles between the struts of the frame can be selected to limit radial expansion of the frame at a given opening pressure (e.g., inflation pressure of the balloon). In particular embodiments, these angles are at least 110 degrees or greater when the frame is expanded to its functional size, and even more particularly these angles are at least 120 degrees or greater when the frame is expanded to its functional size. U.S. Patent Publication No. 2012/0123529 further describes the frame 12 as well as other configurations for frames that can be incorporated in a prosthetic heart valve.

As shown in FIGS. 1-2, the skirt assembly 15 can include an inner skirt 16 that is located inside the frame 12 and an outer skirt 18 that is located outside the frame 12. The outer skirt 18 can include a plurality of circumferentially spaced apart extension portions or projections 66 and recesses 68 between adjacent projections formed along the outflow edge (the upper edge in the illustrated embodiment) of the outer skirt. In other embodiments, the outer skirt 18 can have a straight outflow edge without any projections or recesses (e.g., the outer skirt 202 of FIG. 14).

The inflow (lower) and the outflow (upper) edges of the outer skirt 18 can be secured to the frame 12 and/or the inner skirt 16 by, for example, heat bonding, adhesive, and/or suturing. As shown in the illustrated embodiment, the projections 66 along the outflow edge of the outer skirt 18 can be secured to struts of the frame with sutures 70 while the recesses 68 between adjacent projections can be left unattached to the frame 12 and the inner skirt 16. The outer skirt 18 functions as a sealing member for the prosthetic valve 10 by sealing against the tissue of the native valve annulus, helping to reduce paravalvular leakage past the prosthetic valve 10.

In some embodiments, as shown in FIGS. 1-2, the outer skirt 18 can be configured to extend radially outward from the frame 12 when the prosthetic valve 10 is in a radially expanded configuration. Alternatively, the outer skirt 18 can be configured to form a snug fit with the frame 12 such that it lies against the outer surface of the frame 12 when the prosthetic valve 10 is in the radially expanded configuration (e.g., the outer skirt 202 of FIG. 14). The outer skirt 18 can be formed from any of various synthetic materials or natural tissue (e.g., pericardial tissue). Suitable synthetic materials include any of various biocompatible fabrics (e.g., PET fabric) or non-fabric films, including any of the materials disclosed below for the reinforcing layer 88 of the inner skirt 16. Further details of the outer skirt 18 are also disclosed in U.S. Patent Publication No. 2012/0123529.

As further shown in FIGS. 1-2, the inner skirt 16 in the illustrated embodiment extends from the inlet end 54 of the frame to the fourth row IV of angled struts 28. In other embodiments, the inner skirt 16 can extend from the inlet end 54 of the frame to a location short of the fourth row IV of struts (e.g., to the second row II or the third row III of struts), or the inner skirt can extend the entire height of the frame 12 (e.g., from the inlet end 54 to the outlet end 56). In alternative embodiments, the inner skirt 16 can be positioned and/or sized to extend over a different portion of the frame 12 than the configuration shown in FIGS. 1-2. For example, in some embodiments, the inflow end of the inner skirt 16 can be axially spaced from the inlet end 54 of the frame 12.

Although the inner skirt 16 is typically tubular or cylindrical in shape (forming a complete circle in a cross-sectional profile in a plane perpendicular to the longitudinal axis of the valve), the inner skirt 16 need not extend along the inner surface of the frame 12 in the circumferential direction through 360 degrees. In other words, the inner skirt 16 can have a cross-sectional profile (in a plane perpendicular to the axis of the lumen of the valve) that is not a complete circle. The inner skirt 16 can be initially formed as a flat strip, and then formed to the annular shape by coupling together opposing edge portions, for example, by sewing, thermal bonding, and/or adhesive. Alternatively, the inner skirt 16 can be formed directly in an annular shape, for example, by constructing the inner layer 16 on a cylindrically shaped mandrel as described below.

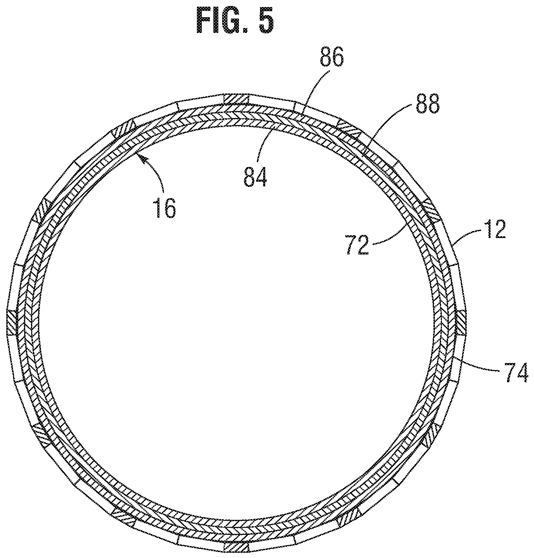

Referring to FIG. 5, the inner skirt 16 has a first side 72 defining an inner surface of the inner skirt and a second side 74 defining an outer surface of the inner skirt 16. As described more fully below, the frame-facing side 74 of the inner skirt 16 can have one or more windows or openings, through which an otherwise encapsulated fabric layer is exposed. Sutures can be threaded through the fabric layer of the inner skirt 16 at those windows to secure the inner skirt 16 to the frame 12. The outer skirt 18 is omitted in FIG. 5 for purposes of illustration.

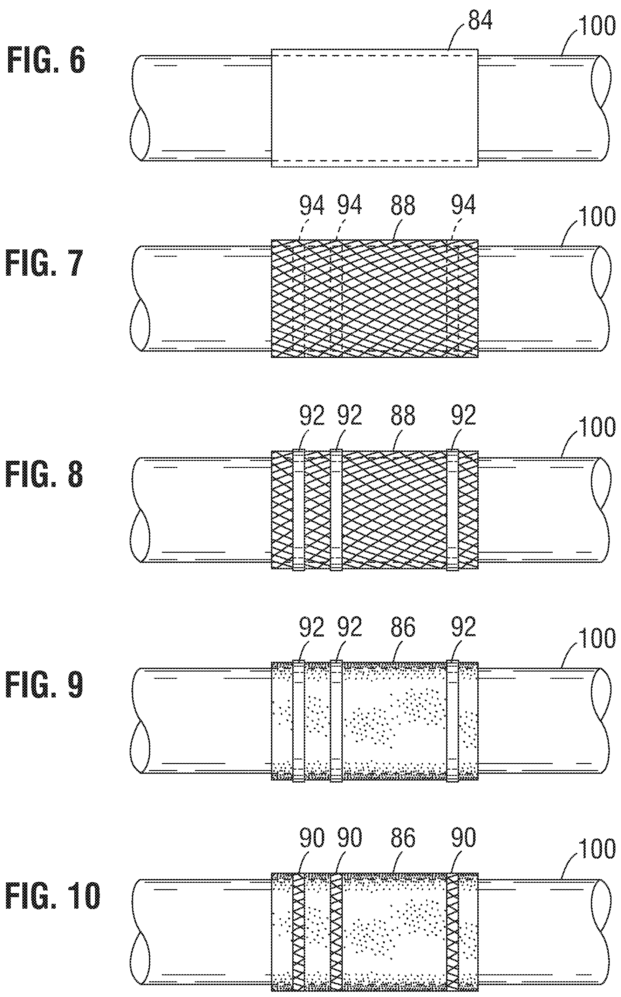

When the inner skirt 16 is mounted to the frame 12, the first side 72 of the inner skirt 16 faces inwardly toward the leaflet structure 14 located interior of the prosthetic valve 10, and the second side 74 of the inner skirt 16 faces outwardly against the inner surface of the frame 12. In particular embodiments, the inner skirt 16 can comprise a reinforcing layer 88 sandwiched between a first covering member 84 and a second covering member 86. In a representative embodiment, the reinforcing layer 88 can be a fabric layer. The first and second covering members 84, 86 can also be referred to as encapsulating layers and form the inner and outer layers, respectively, of the illustrated inner skirt 16. In particular embodiments, the inner surface of the reinforcing layer 88 can be completely covered by the first covering member 84 on the first side 72, and the outer surface of the reinforcing layer 88 is partially covered by the second covering member 86 on the second side 74, with the second covering member 86 defining one or more windows or openings 90 (see FIG. 10) that expose the reinforcing layer 88 on the second side 74.

The reinforcing layer 88 can strengthen the inner skirt 16 to resist tearing. It can also serve as an anchor layer for suturing the inner skirt 16 to the frame 12 and for supporting the cusp edge portions of the leaflets 40, as described more fully below. In addition, the reinforcing layer 88, in cooperation with the encapsulating layers 84, 86, can help decrease (or prevent) paravalvular leakage past the prosthetic valve 10 when in the expanded configuration.

In some embodiments, the reinforcing layer 88 can comprise a woven fabric that is woven from various types of natural or synthetic fibers (or filaments, or yarns, or strands), including but are not limited to: gauze, PET fibers (e.g., Dacron), polyester fibers, polyamide fibers, ultrahigh molecular weight polyethylene (UHMWPE) fibers, etc. In certain embodiments, the reinforcing layer 88 can have a knitted or braided structure rather than the woven structure. Additionally, the reinforcing layer 88 can be a woven, knitted, or braided structure formed from metal filaments or wires (e.g., Nitinol, stainless steel, or titanium filaments or wires), glass filaments or wires, carbon filaments or wires, or ceramic (e.g., alumina oxide) filaments or wires. Alternatively, the reinforcing layer 88 can comprise filaments, fibers, yarns, or wires made of any of the materials described above, where filaments, fibers, yarns, or wires are not necessarily interwoven, braided, or knitted together. For example, the reinforcing layer 88 can comprise a layer of parallel filaments, fibers, yarns, or wires, or layers of filaments, fibers, yarns, or wires laid on top of each other. In certain embodiments, the reinforcing layer 88 can include any of various non-woven fabrics, such as felt. The thickness of the reinforcing layer 88 can vary, but can be less than 6 mil, and desirably less than 4 mil, and even more desirably about 2 mil.

Alternatively, the reinforcing layer 88 can include one or more layers or films formed from any of various semi-crystalline polymeric materials or thermoplastics having aligned or partially aligned (e.g., parallel) molecular chains. Such materials can exhibit anisotropic mechanical properties, such as increased mechanical strength along the longitudinal direction of the molecular chains. Suitable semi-crystalline polymeric materials can include, for example, PTFE, PET, polypropylene, polyamide, polyetheretherketone (PEEK), etc., layers or films of which can be situated between and encapsulated by the encapsulating layers 84, 86, to reinforce the inner skirt 16. Unless otherwise specified, a fabric layer is described in the following description as an exemplary reinforcing layer for illustration purposes, while it is to be understood that a non-fabric layer having sufficiently high tensile strength can also be used as the reinforcing layer.

The encapsulating layers 84, 86 can be made of any suitable biocompatible material. Desirably, the encapsulating layers 84, 86 comprise a material that is relatively less abrasive than the fabric layer to reduce abrasion of the leaflets 40. The encapsulating layers 84, 86 can comprise, for example, a membrane or film formed from non-woven fibers or a non-fibrous material. The biocompatible material used to form the layers 84, 86 may be a non-absorbable polymeric material (i.e., a material that does not dissolve once implanted in the body), and the material may be elastomeric. In addition, any of the encapsulating layers 84, 86 can have a porous microstructure that promotes ingrowth of surrounding tissue to assist in securing the prosthetic valve 10 in a body lumen.

Examples of encapsulating layer materials include, without limitation, ePTFE, unexpanded porous PTFE, polyester or expanded PTFE yarns, PTFE, ultrahigh molecular weight polyethylene (UHMWPE), other polyolefins, composite materials such as ePTFE with PTFE fibers, or UHMWPE film with embedded UHMWPE fibers, polyimides, silicones, polyurethane, hydrogels, fluoroethylpolypropylene (FEP), polypropylfluorinated amines (PFA), other related fluorinated polymers, or various combinations of any of these materials. In particular embodiments, the encapsulating layers 84, 86 can be formed from respective tubes made of a suitable polymeric material (e.g., ePTFE tubes or UHMWPE tubes) that can be bonded to each other when subjected to heat treatment. In some embodiments, the encapsulating layers 84, 86 can be formed from the same type of materials, although different materials can be used to form the encapsulating layers depending on the particular application.

Microporous ePTFE tubes can be made by a number of well-known methods. Expanded PTFE is frequently produced by admixing particulate dry polytetrafluoroethylene resin with a liquid lubricant to form a viscous slurry. The mixture can be poured into a mold, typically a cylindrical mold, and compressed to form a cylindrical billet. The billet can then be ram extruded through an extrusion die into either tubular or sheet structures, termed extrudates in the art. The extrudates comprise an extruded PTFE-lubricant mixture called "wet PTFE." Wet PTFE has a microstructure of coalesced, coherent PTFE resin particles in a highly crystalline state. Following extrusion, the wet PTFE can be heated to a temperature below the flash point of the lubricant to volatilize a major fraction of the lubricant from the PTFE extrudate. The resulting PTFE extrudate without a major fraction of lubricant is known in the art as dried PTFE. The dried PTFE can then be either uniaxially, biaxially, or radially expanded using appropriate mechanical apparatus known in the art. Expansion is typically carried out at an elevated temperature, e.g., above room temperature but below 327°C, the crystalline melt point of PTFE. Uniaxial, biaxial, or radial expansion of the dried PTFE causes the coalesced, coherent PTFE resin to form fibrils emanating from nodes (regions of coalesced PTFE), with the fibrils oriented parallel to the axis of expansion. Once expanded, the dried PTFE is referred to as expanded PTFE ("ePTFE") or microporous PTFE.

UHMWPE is made up of very long chains of polyethylene, with molecular weight numbering in the millions, usually between 2 and 6 million. It is highly resistant to corrosive chemicals, has extremely low moisture absorption and a very low coefficient of friction. It is self-lubricating and highly resistant to abrasion. UHMWPE is processed using compression molding, ram extrusion, gel spinning, and sintering. UHMWPE is available commercially as a powder, in sheets or rods, and as fibers.

The encapsulating layers 84, 86 can be formed by a number of means. For example, in one example, the encapsulating layers 84, 86 can be formed using an electrospinning process, which uses electric force to draw charged threads of polymer solutions or polymer melts up to fiber diameters in the order of some hundred nanometers. In another example, the encapsulating layers 84, 86 can be formed using the centrifugal spinning technique. In centrifugal spinning, the spinning fluid is placed in a rotating spinning head. When the rotating speed reaches a critical value, the centrifugal force overcomes the surface tension of the spinning fluid to eject a liquid jet from the nozzle tip of the spinning head. The jet then undergoes a stretching process and is eventually deposited on the collector, forming solidified nanofibers. Yet in a further example, the encapsulating layers 84, 86 can be formed using an atmospheric plasma spray (APS) technique, which is a special variation of the thermal spray process. APS uses an electric arc to ionize flowing process gases, the hot gas stream can be controlled to melt a very wide range of powder feedstock materials to apply high-quality coatings to a target object. In other embodiments, the encapsulating layers 84, 86 can be formed using any other suitable method including, such as dip coating, spray coating, or melt-spinning. For example, any one of the encapsulating layers 84, 86 can be formed by dipping the fabric layer 88 in a liquefied polymeric material, and then allowing the liquefied polymeric material to cure.

FIGS. 6-10 show one exemplary process of forming the inner skirt 16. Although the use of electrospinning is described below, it is exemplary in nature and is not intended as a limitation. It is to be understood that other processes for depositing a polymeric layer, such as centrifugal spinning, APS, dip coating, and others as described above, can also be used.

First, as depicted in FIG. 6, a first covering member 84 comprising a first coating material can be deposited circumferentially around the outer surface of a cylindrically shaped mandrel 100 by means of eletrospinning (or using other techniques). As known in the art, the electrospinning system can include a spinneret that is used to extrude a polymer solution or polymer melt to form fibers. To deposit the first covering member 84 over the mandrel 100, the electrospinning system can be configured to rotate the fiber-extruding spinneret in a circular motion around the mandrel 100. Alternatively, the fiber-extruding spinneret can be configured to be stationary while the mandrel 100 is placed in front of the spinneret and rotates about its longitudinal axis.

Second, as depicted in FIG. 7, the fabric layer 88 can be placed over the first covering member 84. The fabric layer 88 can be in the form of a sheet of fabric material that is tightly wrapped around the first covering member 84. For example, as described above, the fabric layer 88 can have a woven structure that includes threads of warp and weft fibers extending perpendicular to each other. In alternative embodiments, the fabric layer 88 may also be deposited over the first covering member 84. It should be understood that this method of construction is not limited to embodiments where the reinforcing layer is a woven fabric; this method can be used for forming a skirt wherein the reinforcing layer can take any of the forms disclosed herein. For example, as described above, the fabric layer 88 can include a non-woven fabric, which itself can be formed by means of electrospinning and which desirably has a relatively higher tensile strength than the layers 84, 86. In another example, layer 88 can be a pre-formed braided material that is wrapped around the first covering member 84. In yet another example, layer 88 can be formed by braiding one or more yarns or filaments around the first covering member 84 to form a braided layer around the first covering member.

Third, as depicted in FIG. 8, one or more masks 92 can be placed over the fabric layer 88 at selected areas 94 of the fabric. Fourth, as depicted in FIG. 9, a second covering member 86 comprising of a second coating material can be deposited over the masked fabric layer 88 by means of eletrospinning (or using other techniques). Next, as depicted in FIG. 10, the masks 92 are removed after the deposition of the second covering member 86. Accordingly, one or more windows 90 corresponding to the selected areas 94 are created such that the windows 90 expose the underlying fabric layer 88.

In the embodiment shown FIGS. 8-9, the masks 92 can temporarily cover selected areas 94 of the fabric layer 88, and prevent those selected areas 94 from being deposited by the second coating material of the second covering member 86. Alternatively, the selected areas 94 may be functionally masked without the need of applying the physical masks 94. For example, the relevant movement and operation (e.g., activation and/or deactivation) of the fiber-extruding spinneret with respect to the mandrel 100 may be programmed so that the second coating material of the second covering member 86 can only be deposited on portions of the fabric layer 88 that are outside the selected areas 94.

In the embodiment depicted in FIGS. 8-9, three annular bands of masks 92 are shown, respectively corresponding to three selected areas 94 along the outer circumference of the fabric layer 88. As a result, three annular windows 90 are created after the removal of the masks 92, as depicted in FIG. 10. In other embodiments, any one of the masks 92 can have a non-annular shape, so that the corresponding selected area 94 and the resulting window 90 do not completely encircle the fabric layer 88. For example, any one of the masks 92 may have a customized shape at a customized location so as to create a customized window 90. In addition, although three windows 90 are shown in the illustrated embodiment, the inner skirt can be formed with a fewer or greater number of windows and the windows can be positioned at any locations along the skirt. For example, in some embodiments, the inner skirt can be formed with one or more circumferentially extending rows of windows, with each row comprising of a plurality of circumferentially spaced-apart windows. In other embodiments, one or more windows can be formed along the inlet and/or the outlet edge of the inner skirt.

Although not shown, it is to be understood that in each of the steps described above, an anchoring mechanism can be provided to temporarily secure the position of the each layer. As a non-limiting example, layers of PTFE tape can be wrapped around one or both ends of the second covering member 86 to help secure the position of the second covering member 86 to the underlying layers of the assembly and to the mandrel 100 during subsequent processing.

In a representative embodiment, the fabric layer 88 has a plurality of openings that allow the first covering member 84 and the second covering member 86 to fuse together through those openings. In one example, the openings in a fabric layer 88 may be created by weaving, braiding, or knitting fibers or yarns to form the fabric layer. In another example, the fabric layer 88 may have a non-woven, porous structure with openings. In another example, such as when a non-woven fabric (e.g., a felt) is used to form the fabric layer, openings in the fabric layer 88 can be formed by cutting (e.g., laser cutting) openings in the fabric layer.

In one exemplary embodiment, fusion between the first and second covering members 84, 86 through openings in the fabric layer 88 can occur simultaneously during the process of depositing the second covering member 86 over the masked fabric layer 88. As the second coating material extruded from the spinneret is deposited on the fabric layer 88 to form the second covering member 86, some of the second coating material may penetrate through those openings in the fabric layer 88, and fuse with the fibers in the first covering member 84.

In other embodiments, fusion between the first and second covering members 84, 86 may occur after the deposition of the second covering member 86 over the masked fabric layer 88. For example, the assembly shown in FIG. 10 can undergo an encapsulation process whereby the assembly is subjected to heat and/or pressure to cause the first and second covering members 84, 86 to bond to each other through the openings in the fabric layer 88. In addition, the fabric layer 88 may have an axial length that is shorter than the first and second covering members 84, 86 to facilitate bonding of the first and second covering members 84, 86 at their respective ends to encapsulate the fabric layer 88 therebetween. A similar encapsulation process is described in U.S. Patent Publication Nos. 2014/0209238 and 2016/0317305.

In an exemplary embodiment, ePTFE can be used as the first coating material for depositing the first covering member 84 and/or the second coating material for depositing the second covering member 86. Alternatively, other materials such as UHMWPE, polyurethane composite materials, or any other non-absorbable polymeric materials described above can be used. The inner skirt 16 desirably can have a laminate structure in which the fabric layer 88 is sandwiched between the two fused layers: the first covering member 84 and and second covering member 86. In some embodiments, the same material can be used for depositing the first and second covering members 84, 86. Due to the inter-layer fusion or bonding, the first and second covering members 84, 86 can be merged together, effectively creating a unitary structure (i.e., there is no physical inter-layer boundary), in which the fabric layer 88 is encapsulated. The density of the first covering member 84 can be the same as or different from the density of the second covering member 86. In other embodiments, the first coating material used for depositing the first covering member 84 can be different from the second coating material used for depositing the second covering member 86.

After the first and second covering members 84, 86 are firmly fused together to encapsulate the fabric layer 88, the inner skirt 16 can be removed from the mandrel 100. One or both end portions of the inner skirt 16 can be trimmed to achieve the desired height of the inner skirt. The inner skirt 16 can then be mounted to the frame 12.

Although FIGS. 6-10 and the description above illustrate the process of forming an annularly shaped inner skirt 16, it is to be understood that the same process can be used to form the outer skirt 18. Further, as described above, the inner skirt 16 may be initially formed as a flat strip, and then formed to the annular shape by coupling together its two opposing edges. To form a flat strip, the first and second covering members 84, 86 as well as the fabric layer 88 may be constructed on a flat substrate, instead of the cylindrically shaped mandrel 100 as described above.

Although the process described above uses masking to create the windows 90 on the second covering member 86 of the inner skirt 16, it is to be understood that other methods can be used to create those windows 90. For example, the second covering member 86 can be initially deposited over the entire surface of the fabric layer 88. Then selected areas 94 on the second covering member 86 can be located and removed, for example, by means of laser cutting, chemical erosion, or other means. As a result, windows 90 can be created at the selected areas 94 on the second covering member 86, exposing the underlying fabric layer 88 therein. In another example, the second covering member 86 can be pre-fabricated so that it is devoid of the second coating material in the selected areas 94. Then the pre-fabricated second covering member 86 can be wrapped around the fabric layer 88. As a result, the fabric layer 88 can be exposed through windows 90 created at the selected areas 94. Then, the assembly (the first covering member 84, the fabric layer 88, and the second covering member 86) can undergo the heat- and/or pressure-based encapsulation process as described above, causing the first and second covering members 84, 86 to bond to each other.

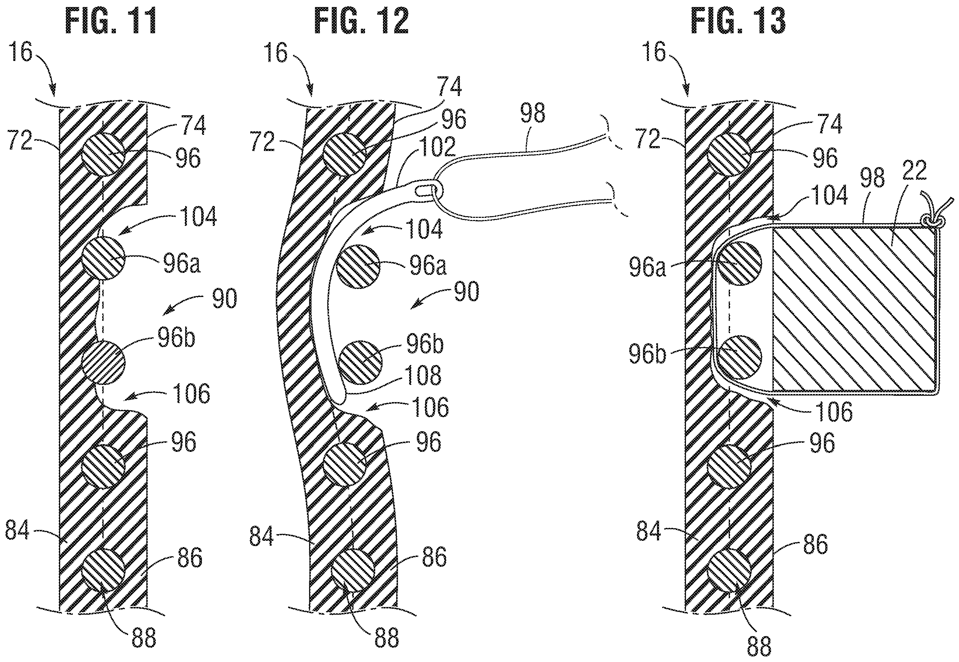

The inner skirt 16 can be sutured to the frame 12 at the locations of the windows 90. For example, the inner skirt 16 can be placed on the inside of frame 12. The positions of the windows 90 can be arranged so as to generally correspond to the first, third, and fourth rows of struts 22, 26 and 28, respectively, although other configurations can be used. The inner skirt 16 can also be secured to the struts of the first, third, and fourth rows of struts with sutures extending around the struts and through the fabric layer 88 at the locations of the windows 90, as further described below in connection with FIGS. 11-13. Since the windows in the illustrated embodiment extend continuously around the entire circumference of the inner skirt, a continuous, circumferentially extending whip stitch can be formed along each row of struts and the fabric layer 88 at each window 90.

As described above, the windows 90 can be created at selected locations and can have any of various shapes, allowing the inner skirt to be sutured to the frame at different locations. For example, as noted above, the inner skirt can be formed with rows of circumferentially spaced-part windows, which can allow placement of individual sutures or stitching that does not extend continuously along an entire row of struts, such as at selected portions on the inner skirt that are more prone to tension or stress.

FIGS. 11-13 illustrate an exemplary method of suturing the inner skirt 16 to the frame 12. FIG. 11 shows a cross-sectional view of a portion of an inner skirt 16 having a first side 72 and a second side 74. As described above, the inner skirt 16 has an encapsulated fabric layer 88 sandwiched between the first covering member 84 on the first side 72 and the second covering member 86 on the second side 74. FIG. 11 also depicts a window 90 on the second covering member 86 of the inner skirt 16, exposing the underlying fabric layer 88. The inter-layer boundary (as indicated by the dashed line) may be absent when the same material is used to form the encapsulating layers 84, 86, which can be fused or bonded together to form a single, unitary structure encapsulating the fabric. In the depicted embodiment, the fabric layer 88 is shown to have a woven structure comprising woven filaments, fibers, or yarns 96. The woven filaments 96 desirably have sufficient strength to serve as anchors to retain sutures 98, as described below.

FIG. 12 schematically shows how to thread a suture 98 between the fabric layer 88 and the first covering member 84 through the window 90. In the depicted embodiment, the suture 98 is attached to a needle 102. The tip 108 of the needle 102 is desirably blunted. By sliding the needle 102 from the second side 74 and through the window 90 while applying light force at the needle tip 108, the first covering member 84 can be slightly pushed away from the fabric layer 88, thus creating a space for the needle 102 to be inserted between the first covering member 84 and the fabric layer 88. As shown, the needle 102 and the attached suture 98 can slide into the fabric layer 88 from a first end 104 of the window 90, pass behind one or more filaments 96 that are exposed by the window 90 (e.g., 96a and 96b), and then slide out of fabric layer 88 at a second end 106 of the window 80. In this manner, the suture 98 does not extend through the entire thickness of the inner skirt 16. In some embodiments, the first covering member 84 may not separate from the fabric layer 88 by insertion of the needle 102 (as depicted in FIG. 12), in which case the needle 102 and the attached suture 98 may be threaded partially through the thickness of the first covering member 84 but does not extend through the entire thickness of the inner skirt.

FIG. 13 schematically illustrates suturing the fabric layer 88 of the inner skirt 16 to an adjacent strut 22 of the frame 12. Desirably, the first side 72 of the inner skirt 16 faces inwardly toward the leaflet structure 14 located interior of the prosthetic valve 10, and the second side 74 of the inner skirt 16 faces outwardly against the frame 12. By threading the needle 102 and the attached suture 98 between the fabric layer 88 and the first covering member 84 through the window 90, the woven filaments 96 between the first and second ends 104, 106 of the window 80 (e.g., 96a and 96b) can collectively serve as anchors to hold the suture 98. The suture 98 can then be wrapped around the adjacent strut 22, thus securing those woven filaments (e.g., 96a and 96b) to the adjacent strut 22. Accordingly, the inner skirt 16 can be securely attached to the frame 12. FIG. 13 depicts a strut 22 for purposes of illustration. It should be understood that the inner skirt 16 can be sutured to other struts of the frame (e.g., 26, 28, 32) in a similar manner.

As described above, the fabric layer 88 may also have a non-woven structure that has no distinct woven threads 96. In such case, the suture 98 may be attached to a needle that has a pointed tip. The needle can be used to pierce the fabric layer 88 and thread the suture 98 through the fabric layer. In this manner, the portion of the fabric layer 88 that is between the first and second ends 104, 106 of the window 90 can function as an anchor to hold the suture 98, which in turns secures that portion of the fabric to the adjacent strut 22. Accordingly, the inner skirt 16 can be securely attached to the frame 12.

Because the suture 98 is routed between the first covering member 84 and the fabric layer 88, it is not exposed on the first side 72 of the inner skirt 16. In other words, the suture 98 is covered by the first covering member 84. The inner surface of the fabric layer is also covered by the first covering member. Therefore, abrasion of leaflets 40 due to repetitive contact between the leaflets 40 and inner skirt 16 and between the leaflets 40 and the sutures 98 during working cycles of the prosthetic valve 10 can be avoided. Desirably, the inner skirt 16 is sutured to the frame 12 only at the one or more windows 90 on the second covering member 86, so that contact between the moveable portions of the leaflets 40 and the sutures 98 can be avoided. Additionally, the first covering member 84 desirably covers the entire extent of the inner surface of the fabric layer, or at least the portions of the fabric layer that would otherwise contact the moveable portions of the leaflet during working cycles of the prosthetic valve. In some embodiments, sutures 98 may pass through the entire thickness of the inner skirt, such as at locations on the inner skirt that would not come in contact with moveable portions of the leaflets.

As noted above, the leaflets 40 can be secured to one another at their adjacent sides to form commissures 58. Each commissure 58 can be secured to a corresponding commissure window 20 of the frame 12, as described in U.S. Patent Publication No. 2012/0123529. The inflow or cusp edges 52 of the leaflets 40 can be sutured to the inner skirt 16 along a suture line that tracks the curvature of the scalloped inflow edge of the leaflet structure. The fabric layer 88 can provide the strength required to retain the sutures. Any suitable suture, such as an Ethibond suture, can be used to secure the leaflets 40 to the fabric layer 88 of the inner skirt.

In some embodiments, the inflow edges 52 of the leaflets 40 are secured to the inner skirt 16 prior to mounting the inner skirt 16 to the frame. After securing the leaflets 40 to the inner skirt 16, the inner skirt is then secured to the frame as described above and the commissures 58 of the leaflets are mounted to the frame. In other embodiments, the inner skirt 16 can be mounted to the frame without the leaflets, after which the inflow edges 52 of the leaflets are then secured to the inner skirt.

In certain embodiments, the inflow edges 52 of the leaflets 40 can be secured to the inner skirt via a thin PET reinforcing strip (not shown), as disclosed in U.S. Pat. No. 7,993,394. As described in U.S. Pat. No. 7,993,394, the reinforcing strip can be sutured to the inflow edges of the leaflets. The reinforcing strip and the lower edges of the leaflets can then be sutured to the inner skirt 16. The reinforcing strip desirably is secured to the inner surfaces of the leaflets 40 such that the inflow edges 52 of the leaflets become sandwiched between the reinforcing strip and the inner skirt when the leaflets and the reinforcing strip are secured to the inner skirt. The reinforcing strip enables a secure suturing and protects the pericardial tissue of the leaflet structure from tears.

As described above, the outer skirt 18 can be constructed in a similar manner as the inner skirt 16. That is, the outer skirt 18 can also have a reinforcing layer (e.g., a fabric layer 88) sandwiched between encapsulating layers 84, 86. Similarly, windows 90 can be created on one of the encapsulating layers 84, 86. Because the outer skirt 18 is attached to the outside of the frame 12, the outer layer 18 is desirably arranged so that the frame 12 faces the side of the outer skirt 18 that has the windows 90. In such arrangement, the outer skirt 18 can be attached to the frame 12 by suturing the encapsulated fabric layer 88 to the frame 12 through the frame-facing windows 90.

Yet in another embodiment, the outer skirt 18 can have the fabric layer 88 being coated with only one of the encapsulating layers 84, 86. While attaching the outer skirt 18 to the frame 12, the outer skirt 18 can be arranged so that the uncoated side of the fabric layer 88 faces inwardly toward the frame 12, so that the outer skirt 18 can be attached to the frame 12 by suturing the exposed fabric layer 88 to the frame 12.

Alternatively, the outer skirt 18 can comprise only the fabric layer 88 without any of the encapsulating layers 84, 86. As such, the outer skirt 18 can be directly sutured to the frame 12. Because the sutures on the outer skirt 18 are not subject to repetitive contact by the moving leaflets 40, abrasion of the leaflets due to the sutures on the outer skirt 18 may be less a concern than the sutures on the inner skirt 16. By eliminating one or both encapsulating layers 84, 86, the outer layer 18 may be constructed thinner, thus reducing the overall profile of the valve 10 when it is crimped to a radially compressed state.

FIG. 14 shows a prosthetic valve 200, according to another embodiment. The prosthetic valve 200 can have the same construction and components as the prosthetic valve 10 of FIGS. 1-2, except that the prosthetic valve 200 includes a different outer skirt. Common components in FIGS. 1-2 and FIG. 14 are given the same reference numbers and are not described further.

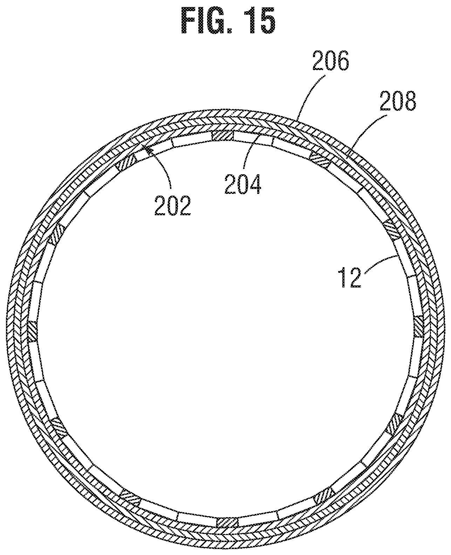

The prosthetic valve 200 includes an outer skirt 202 similar in construction to the inner skirt 16. As such, the outer skirt 202 includes an inner encapsulation layer 204, an outer encapsulation layer 206, and a reinforcing layer 208 disposed between the layers 204, 206. The outer skirt is mounted on the outside of the frame 12. The skirt 202 can be formed and mounted to the frame 12 using any of the techniques described above in connection with the skirt 16. FIG. 15 shows cross-sectional view of the frame 12 and the outer skirt 202 with the other components of the prosthetic valve removed for purposes of illustration. The outer skirt 202 can be configured to form a snug fit with the frame 12 such that it lies against the outer surface of the frame 12 when the prosthetic valve 200 is in the radially expanded configuration, as shown in FIGS. 14-15.

When the prosthetic valve 200 is radially compressed or crimped to a radially compressed state for delivery into a patient's body (e.g., on a balloon of a delivery apparatus), the frame 12 is elongated in the direction of the frame's longitudinal axis L. When the prosthetic valve is radially expanded from the radially compressed state to a radially expanded state, the frame 12 foreshortens axially along the axis L. When the outer skirt is relatively tight or snug fitting around the frame 12, it is desirable that the outer skirt exhibit sufficient elongation or stretchability in the axial direction so as not to inhibit elongation of the frame 12 during crimping of the prosthetic valve.

To such ends, in particular embodiments, the reinforcement layer 208 comprises yarns, filaments, fibers, or wires that are non-perpendicular upper and lower edges 214, 216, respectively, of the skirt 202. Stated differently, the yarns, filaments, fibers, or wires extend at angles greater than 0 degrees and less than 90 degrees relative to the longitudinal axis L of the frame 12 (the yarns, filaments, fibers, or wires are non-parallel and non-perpendicular to the longitudinal axis L of the frame).

In certain embodiments, the reinforcing layer 208 comprises a textile with interlacing yarns, such as a woven, braided or knitted structure. In a specific implementation, the reinforcing layer 208 comprises a fabric, such as a plain weave fabric, having a first set of yarns 210 woven together with a second set of yarns 212, wherein the yarns 210, 212 are non-perpendicular to the upper and lower edges 214, 216, respectively, of the skirt. Stated differently, the yarns 210, 212 extend at angles greater than 0 degrees and less than 90 degrees relative to the longitudinal axis L of the frame 12.

In some examples, the yarns 210, 212 extend at angles in the range of 20 degrees to 70 degrees relative to the upper and lower edges 214, 216, and more desirably in the range of 30 degrees to 60 degrees relative to the upper and lower edges 214, 216, and more desirably in the range of 40 degrees to 50 degrees relative to the upper and lower edges 214, 216. In a specific implementation, the yarns 210, 212 extends at 45 degrees relative to the upper and lower edges 214, 216 and a longitudinal axis L of the prosthetic valve.

In some embodiments, the yarns 210, 212 are parallel to the struts of the frame to which the outer skirt is connected when the prosthetic valve is in the expanded state. In particular embodiments, the skirt 202 is sutured to one or more of the angled struts 22, 24, 26, and 28 (see FIG. 4). The frame struts to which the outer skirt is connected to are also referred to as "skirt-supporting struts." Typically, though not necessarily, the skirt-supporting struts are oriented at 45-degree angles relative to the longitudinal axis L. However, in other embodiments, the skirt-supporting struts and the yarns 210, 212 can be greater or less than 45-degrees relative to the longitudinal axis L, depending on the particular frame configuration.

The fabric layer 208 can be formed by weaving the yarns at the selected angle (e.g., 45 degrees) relative to the upper and lower edges of the fabric. Alternatively, the fabric layer 208 can be diagonally cut from a vertically woven fabric (where the yarns extend perpendicular to the edges of the material) such that the fibers extend at a selected angles (e.g., 45 degrees) relative to the cut upper and lower edges of the fabric. The yarns 210, 212 can comprise multi-filament yarns (yarns comprising plural fibers or filaments) or monofilament yarns (yarns comprising single fibers or filaments).

In some embodiments, the frame 12 can be partially crimped during assembly of the prosthetic valve so that the skirt-supporting struts are parallel to the yarns 210, 212 in the partially crimped state. The skirt 202 can then be mounted to the frame in the partially crimped state, such as via any of the techniques described herein.

Due to the orientation of the yarns relative to the upper and lower edges, the fabric layer can undergo greater elongation in the axial direction (i.e., in a direction from the upper edge 214 to the lower edge 216 parallel to axis L). Thus, when the metal frame 12 is crimped, the skirt 202 can elongate in the axial direction along with the frame and therefore provides a more uniform and predictable crimping profile. Each cell of the metal frame in the illustrated embodiment includes at least four angled struts (e.g., struts 22, 24, 26) that rotate towards the axial direction (i.e., the angled struts become more aligned with the length of the frame). The angled struts of each cell function as a mechanism for rotating the yarns 210, 212 of the fabric layer 208 in the same direction of the struts, allowing the skirt 202 to elongate along the length of the struts. This allows for greater elongation of the skirt and avoids undesirable deformation of the struts when the prosthetic valve is crimped. As described above, the encapsulation layers 204, 206 can be made of any various elastomers described above in connection with layers 84 and 86, such as silicone or polyurethane, that can stretch axially when the prosthetic valve is crimped and the frame is elongated.

The movement of the yarns 210, 212 during crimping is illustrated in FIGS. 16A and 16B. FIG. 16A shows portions of two interwoven yarns 210, 212 when the outer skirt 202 is in a relaxed state, corresponding to the radially expanded state of the prosthetic valve 200. FIG. 16B shows the yarns 210, 212 when the outer skirt 202 is in an axially stretched or elongated state, corresponding to the radially compressed state of the prosthetic valve after crimping. As shown, the yarns 210, 212 move from a 45-degree angle orientation relative to the longitudinal axis L toward an orientation where the yarns are closer to parallel with the longitudinal axis L.

In addition, the spacing between the woven (or braided or knitted) yarns can be increased to facilitate elongation of the skirt 202 in the axial direction. For example, in particular embodiments, the fabric layer 208 can have a weave density less than 150 ppi (picks per inch), more desirably less than 100 ppi, more desirably less than 70 ppi, and even more desirably 50 ppi or less. In certain embodiments, the fabric layer 208 can have a weave density of about 30 to about 50 ppi, as compared to a known fabric skirts that have a weave density of about 150 to 160 ppi. This construction can allow the skirt 202 to stretch or elongate axially up to at least 40% of its initial length D when the prosthetic valve is radially compressed (the initial length D being the distance between the upper and lower edges 214, 216 of the skirt when the prosthetic valve is in the radially expanded state). At such low weave densities, textiles with interlacing yarns (e.g., woven, braided or knitted structures) can be unstable and tend to unravel. Advantageously, the encapsulation layers 204, 206 encapsulate the yarns and serve as a support mechanism that prevents unraveling of the relatively loose weave. Moreover, during the assembly process described above in connection with FIGS. 6-10, the first encapsulation layer (layer 84 in FIGS. 6-10) can adhere to the textile reinforcing layer (layer 88), which helps stabilize the textile and can avoid unraveling until the second encapsulation layer (layer 86) is formed or placed over the textile reinforcing layer. In addition, as noted above, the textile reinforcing layer in some embodiments can be braided around the first encapsulation layer supported on a mandrel 100, which further helps stabilize the braid.

In certain embodiments, the yarns 210, 212 can have about 10 to about 50 filaments per yarn, with 20 filaments per yarn being a specific example. The filaments of the yarns 210, 212 can have a thickness in the range of about 8 microns to about 16 microns, with 10 microns being a specific example. The filaments of the yarns 210, 212 can be made of PET or UHMWPE, although the filaments can be made of any of the materials described above in connection with the reinforcing layer 88.

In particular embodiments, the yarns 210, 212 can be texturized to increase the elasticity of the skirt in the axial direction. For example, the yarns can be bulked, wherein for example, the yarns are twisted or coiled, heat set, and untwisted or uncoiled such that the yarns retain their deformed (e.g., twisted or coiled) shape in the relaxed or non-stretched configuration. When the prosthetic valve is radially compressed and the skirt 202 is stretched axially, the yarns 210, 212 can straighten from their deformed (e.g., twisted or coiled) state to increase the elasticity and elongation of the skirt. Fabrics comprising texturized yarns are further disclosed in U.S. Publication No. 2018/0206982. Since the yarns 210, 212 can be embedded within elastomeric encapsulation layers 204, 206, the encapsulation layers can stretch axially to accommodate the straightening of the yarns 210, 212. Utilizing such texturized yarns 210, 212 can allow the skirt 202 to stretch axially greater than 40% of its initially length D when the prosthetic valve is radially compressed.

In some embodiments, the reinforcing layer 208 can be a fabric as described above, with the addition of one or more leno yarns woven into the fabric to increase the stability of the fabric. Any of various leno weave patterns can be used to weave the leno yarns into the fabric layer 208. Further details regarding fabric skirts that include leno weaves are disclosed in U.S. Publication No. 2019/0192296 and U.S. Application No. 16/521,226, filed July 24, 2019.

In alternative embodiments, the reinforcing layer 208 can have any of the structures and can be made from any of the materials described above in connection with the reinforcing layer 88. Also, the reinforcing layer 208 can be or include any of the textiles disclosed in U.S. Publication No. 2019/0192296 and U.S. Application No. 16/521,226. For example, the reinforcing layer 208 can be a braided layer or a knitted layer, where the braided or knitted layer is formed from yarns, filaments, or wires made of any of the materials described above, including synthetic materials, metals, glass, carbon, or ceramics. Alternatively, the reinforcing layer 208 can comprise filaments, fibers, yarns, or wires made of any of the materials described above, where filaments, fibers, yarns, or wires are not necessarily interwoven, braided, or knitted together. For example, the reinforcing layer 208 can comprise a layer of parallel filaments, fibers, yarns, or wires, or layers of filaments, fibers, yarns, or wires laid on top of each other.

In such embodiments where the reinforcing layer 208 comprises a braided or knitted layer, the yarns, fibers, or wires can be oriented at non-perpendicular angles relative to the upper and lower edges 214, 216 of the skirt 202 (e.g., 45 degrees) to promote elongation of the skirt during crimping of the prosthetic valve. Similarly, where the reinforcing layer is formed from yarns, fibers, or wires that are not interwoven, braided or knitted together, the yarns, fibers, or wires can be oriented at non-perpendicular angles relative to the upper and lower edges 214, 216 of the skirt 202 (e.g., 45 degrees) to promote elongation of the skirt during crimping of the prosthetic valve.

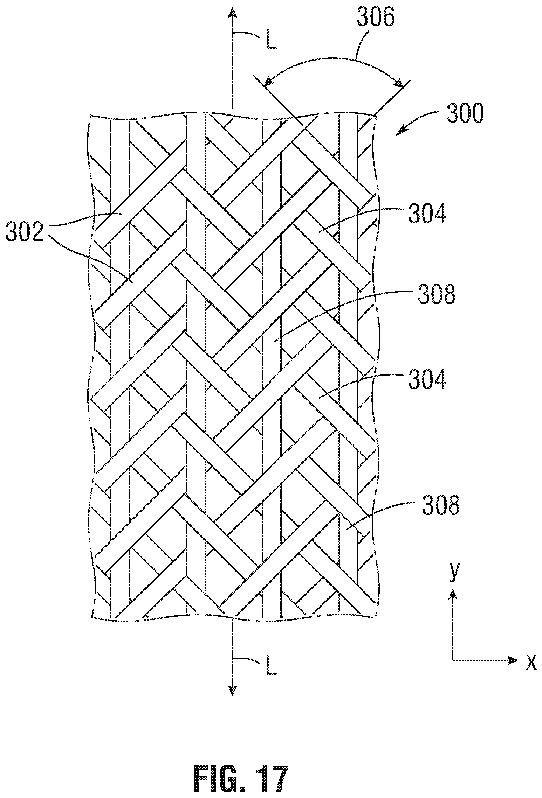

FIG. 17 shows one example of a section of a braided reinforcing layer 300 that can be used in the skirt 202. In FIG. 17, the x-axis represents the circumferential direction of the skirt 202 and the y-axis represents the axial direction of the skirt 202. The braided layer 300 is constructed of first and second sets of yarns 302, 304 braided together, such as in a biaxial braid. FIG. 17 shows the braided layer 300 when the prosthetic valve is radially expanded. This can be a relaxed state of the braided layer, meaning that the braided layer is not stretched or elongated in any direction (or at least not stretched or elongated in the axial direction). As shown, the yarns 302, 304 can be oriented at 45-degree angles with respect to the longitudinal axis L and can form a braid angle 306 of 90 degrees. Thus, in some embodiments, the yarns 302, 304 can extend parallel to the struts of the frame 12 when the frame is in the radially expanded state.

The braided layer 300 can have similar braiding densities as described above for fabric layer 208 to promote elongation of the skirt. As such, the braided layer 300 can have a braid density of less than 150 ppi, more desirably less than 100 ppi, more desirably less than 70 ppi, and even more desirably 50 ppi or less. In certain examples, the braided layer 300 has a braid density of about 30 to about 50 ppi.

To help stabilize the braid, the layer 300 can include a third set of axially extending yarns 308 braided together with yarns 302, 304 to form a triaxial braid. The yarns 308 can be made of an elastomer (e.g., polyurethane (PU), such as a thermoplastic polyurethane (TPU)) that can stretch axially when the prosthetic valve is radially compressed. In lieu of or in addition to forming the yarns 308 from an elastomer, the yarns 308 can be texturized as disclosed above to increase the elasticity of the skirt in the axial direction. The yarns 302, 304 can be made of the same material as the yarns 308 or a different material.

Further, the reinforcing layer 208, 300 can be a hybrid textile (e.g., a woven, braided, or knitted structure) that incorporates yarns or filaments of different materials with different material properties. For example, one or more yarns of the textile can be relatively non-elastic or less elastic than one or more other yarns of the textile. In one implementation, for example, the one or more relatively less elastic yarns can be made of PET and the one or more relatively more elastic yarns can be made of PU, TPU, or other elastomers. The less elastic yarns can be selectively positioned within the textile (according to a predefined pattern) to strengthen the textile while the more elastic yarns can be selectively positioned (according to a predefined pattern) to promote elasticity in a given direction (e.g., in the axial direction). For example, in the embodiment of FIG. 17, the yarns 302, 304 can be made of PET and the yarns 308 can be made of PU or TPU.

It should be understood that the inner skirt 16 can have any of the configurations disclosed above for the outer skirt 202. For example, in some embodiments, the inner skirt 16 of the prosthetic valve 10, 200 similarly can have yarns, fibers, or wires oriented at non-perpendicular angles relative to the upper and lower edges of the skirt 16 (e.g., 45 degrees) to promote elongation of the skirt during crimping of the prosthetic valve.

In some embodiments, the prosthetic valve 200 can have an inner skirt 16 comprising a conventional woven fabric without any encapsulation layers. In other embodiments, the prosthetic valve can be without an inner skirt 16.

### General Considerations

It should be understood that the disclosed embodiments can be adapted to deliver and implant prosthetic devices in any of the native annuluses of the heart (e.g., the pulmonary, mitral, and tricuspid annuluses), and can be used with any of various delivery approaches (e.g., retrograde, antegrade, transseptal, transventricular, transatrial, etc.).

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or", as well as "and" and "or".

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope of these claims.

## Claims

1. An implantable prosthetic valve (200; 300) comprising:
an annular frame (12) that is radially expandable from a radially compressed state to a radially expanded state, the frame (12) having an inflow end, an outflow end, and a longitudinal axis (L) extending from the inflow end to the outflow end;
a plurality of leaflets (40) positioned to regulate a flow of blood from the inflow end to the outflow end of the frame (12); and
a skirt assembly (15) comprising a laminate having a textile layer (208; 300) sandwiched between a first encapsulation layer (206) and a second encapsulation layer (204), wherein the first and second encapsulation layers (204) are made of an elastomer and the textile layer (208; 300) comprises a first set of yarns (210; 302) and a second set of yarns (212; 304) interwoven with the first set of yarns (210; 302), wherein the first and second sets of yarns are non-perpendicular and non-parallel to the longitudinal axis (L);
wherein the textile layer (208; 300) has a weave density of less than 150 ppi, i.e. 150 picks per inch, wherein one inch equals 2,54 centimetres.

2. The prosthetic valve (200) of claim 1, wherein the textile layer (208; 300) has a weave density of 50 ppi or less; preferably wherein the textile layer (208; 300) has a weave density from about 30 to about 50 ppi.

3. The prosthetic valve (200) of any previous claim, wherein the skirt assembly (15) comprises an outer skirt (202) that is mounted to an outer surface of the frame (12).

4. The prosthetic valve (200) of any of claims 1 to 3, wherein the skirt assembly (15) comprises an inner skirt (16) that is mounted to an inner surface of the frame (12).

5. The prosthetic valve (200) of any previous claim, wherein the textile layer (208; 300) is a braided layer.

6. The prosthetic valve (200) of any previous claim, wherein the yarns of the first and second sets of yarns are oriented at angles in the range of 20 to 70 degrees, in the range of 30 to 60 degrees, in the range of 40 to 50 degrees or at 45-degree angles relative to the longitudinal axis (L) of the frame (12).

7. The prosthetic valve (200) of any previous claim, wherein the yarns of the first and second sets of yarns have about 10 to about 50 filaments per yarn; preferably wherein the yarns of the first and second sets of yarns have about 20 filaments per yarn.

8. The prosthetic valve (200) of claim 7, wherein the filaments of the yarns have a thickness ranging from about 8 microns to about 16 microns; preferably wherein the filaments of the yarns have a thickness about 10 microns.

9. The prosthetic valve (200) of any previous claim, wherein at least some of the yarns of the first and second sets of yarns are texturized such that they can retain a twisted or coiled state when the prosthetic valve (200) is in the radially expanded state and are straightened to an untwisted or uncoiled state when the prosthetic valve (200) is in the radially compressed state.

10. The prosthetic valve (200) of any previous claim, wherein the textile layer (208; 300) comprises leno woven yarns.

11. The prosthetic valve (200) of any previous claim, wherein the yarns of the first and second sets of yarns comprise a first type of yarns and a second type of yarns, wherein the first type of yarns are non-elastic or less elastic than the second type of yarns.

12. The prosthetic valve (200) of any previous claim, wherein the textile layer (208; 300) comprises a third set of yarns (308) extending axially and braided together with the first and second sets of yarns to form a triaxial braid.

13. The prosthetic valve (200) of claim 12, wherein the third set of yarns (308) are made of an elastomer that is configured to stretch axially when the prosthetic valve (200) is radially compressed from the radially expanded state to the radially compressed state;
and/or wherein the third set of yarns (308) are texturized such that they can retain a twisted or coiled state when the prosthetic valve (200) is in the radially expanded state and straightened to an untwisted or uncoiled state when the prosthetic valve (200) is in the radially compressed state.

14. The prosthetic valve (200) of any previous claim, wherein when the prosthetic valve (200) is in the radially compressed state, the skirt assembly (15) is elongated axially up to at least 40% of its initial length when the prosthetic valve (200) is in the radially expanded state.

15. A method of assembling a prosthetic valve (200), comprising:
mounting a plurality of leaflets (40) to an annular frame (12) that is radially expandable from a radially compressed state to a radially expanded state, wherein the frame (12) has an inflow end, an outflow end, and a longitudinal axis (L) extending from the inflow end to the outflow end, wherein the plurality of leaflets (40) are configured to regulate a flow of blood from the inflow end to the outflow end of the frame (12); and
mounting a skirt assembly (15) to the annular frame (12), wherein the skirt assembly (15) comprises a laminate having a textile layer (208; 300) sandwiched between a first encapsulation layer (206) and a second encapsulation layer (204), wherein the first and second encapsulation layers (204) are made of an elastomer and the textile layer (208; 300) comprises a first set of yarns (210; 302) and a second set of yarns (212; 304) interwoven with the first set of yarns (210; 302), wherein the first and second sets of yarns are non-perpendicular and non-parallel to the longitudinal axis (L);
wherein the textile layer (208; 300) has a weave density of less than 150 ppi.

## Patentansprüche

1. Implantierbare Klappenprothese (200; 300), umfassend:
einen kranzförmigen Rahmen (12), der radial aus einem radial komprimierten Zustand in einen radial expandierten Zustand expandierbar ist, wobei der Rahmen (12) ein Einströmende, ein Ausströmende und eine längsgerichtete Achse (L) hat, die sich von dem Einströmende zu dem Ausströmende erstreckt;
eine Vielzahl von Segeln (40), die positioniert ist, um einen Blutfluss von dem Einströmende zu dem Ausströmende des Rahmens (12) zu regulieren; und
eine Schürzenanordnung (15), umfassend ein Laminat mit einer Textilschicht (208; 300), die sandwichartig zwischen einer ersten Verkapselungsschicht (206) und einer zweiten Verkapselungsschicht (204) angeordnet ist, wobei die erste und die zweite Verkapselungsschicht (204) aus einem Elastomer gefertigt sind, und die Textilschicht (208; 300) einen ersten Satz von Garnen (210; 302) und einen zweiten Satz von Garnen (212; 304), der mit dem ersten Satz von Garnen (210; 302) verwebt ist, umfasst, wobei der erste und der zweite Satz von Garnen nicht-rechtwinklig und nicht-parallel zu der längsgerichteten Achse (L) sind;
wobei die Textilschicht (208; 300) eine Webdichte von weniger als 150 ppi, i.e. 150 Picks per Inch (Schussfäden pro Zoll), aufweist, wobei ein Inch gleich 2,54 Zentimetern ist.

2. Klappenprothese (200) nach Anspruch 1, wobei die Textilschicht (208; 300) eine Webdichte von 50 ppi oder weniger aufweist; wobei die Textilschicht (208; 300) vorzugsweise eine Webdichte von etwa 30 bis etwa 50 ppi aufweist.

3. Klappenprothese (200) nach einem der vorhergehenden Ansprüche, wobei die Schürzenanordnung (15) eine Außenschürze (202) umfasst, die an einer Außenoberfläche des Rahmens (12) montiert ist.

4. Klappenprothese (200) nach einem der Ansprüche 1 bis 3, wobei die Schürzenanordnung (15) eine Innenschürze (16) umfasst, die an einer Innenoberfläche des Rahmens (12) montiert ist.

5. Klappenprothese (200) nach einem der vorhergehenden Ansprüche, wobei die Textilschicht (208; 300) eine geflochtene Schicht ist.

6. Klappenprothese (200) nach einem der vorhergehenden Ansprüche, wobei die Garne des ersten und zweiten Satzes von Garnen in Winkeln im Bereich von 20 bis 70 Grad, im Bereich von 30 bis 60 Grad, im Bereich von 40 bis 50 Grad oder in 45 Grad Winkeln relativ zu der längsgerichteten Achse (L) des Rahmens (12) orientiert sind.

7. Klappenprothese (200) nach einem der vorhergehenden Ansprüche, wobei die Garne des ersten und zweiten Satzes von Garnen etwa 10 bis etwa 50 Filamente pro Garn aufweisen; wobei die Garne des ersten und zweiten Satzes von Garnen etwa 20 Filamente pro Garn aufweisen.

8. Klappenprothese (200) nach Anspruch 7, wobei die Filamente der Garne eine Dicke im Bereich von etwa 8 Mikrometer bis etwa 16 Mikrometer aufweisen; wobei die Filamente der Garne vorzugsweise eine Dicke von etwa 10 Mikrometer aufweisen.

9. Klappenprothese (200) nach einem der vorhergehenden Ansprüche, wobei mindestens einige der Garne des ersten und zweiten Satzes von Garnen texturiert sind, so dass sie einen verdrehten oder gewendelten Zustand aufrechterhalten können, wenn die Klappenprothese (200) sich in dem radial expandierten Zustand befindet, und zu einem unverdrehten oder ungewendelten Zustand begradigt werden, wenn sich die Klappenprothese (200) in dem radial komprimierten Zustand befindet.

10. Klappenprothese (200) nach einem der vorhergehenden Ansprüche, wobei die Textilschicht (208; 300) leno-gewebte Garne umfasst.

11. Klappenprothese (200) nach einem der vorhergehenden Ansprüche, wobei die Garne des ersten und zweiten Satzes von Garnen einen ersten Typ von Garnen und einen zweiten Typ von Garnen umfassen, wobei der erste Typ von Garnen nichtelastisch oder weniger elastisch als der zweite Typ von Garnen ist.

12. Klappenprothese (200) nach einem der vorhergehenden Ansprüche, wobei die Textilschicht (208; 300) einen dritten Satz von Garnen (308) umfasst, der sich axial erstreckt und mit dem ersten und zweiten Satz von Garnen verflochten ist, um ein triaxiales Geflecht zu bilden.

13. Klappenprothese (200) nach Anspruch 12, wobei der dritte Satz von Garnen (308) aus einem Elastomer gefertigt ist, das ausgestaltet ist, um sich axial zu strecken, wenn die Klappenprothese (200) aus dem radial expandierten Zustand radial zu dem radial komprimierten Zustand komprimiert wird,
und/oder wobei der dritte Satz von Garnen (308) texturiert ist, so dass sie einen verdrehten oder gewendelten Zustand aufrechterhalten können, wenn die Klappenprothese (200) sich in dem radial expandierten Zustand befindet, und zu einem unverdrehten oder ungewendelten Zustand begradigt werden, wenn sich die Klappenprothese (200) in dem radial komprimierten Zustand befindet.

14. Klappenprothese (200) nach einem der vorhergehenden Ansprüche, wobei die Schürzenanordnung (15), wenn sich die Klappenprothese (200) in dem radial komprimierten Zustand befindet, axial bis zu mindestens 40 % ihrer Anfangslänge gelängt wird, wenn sich die Klappenprothese (200) in dem radial expandierten Zustand befindet.

15. Verfahren zum Montieren einer Klappenprothese (200), umfassend:
Montieren einer Vielzahl von Segeln (40) an einem kranzförmigen Rahmen (12), der aus einem radial komprimierten Zustand radial zu einem radial expandierten Zustand expandierbar ist, wobei der Rahmen (12) ein Einströmende, ein Ausströmende und eine längsgerichtete Achse (L) aufweist, die sich von dem Einströmende zu dem Ausströmende erstreckt, wobei die Vielzahl von Segeln (40) ausgestaltet ist, um einen Blutfluss von dem Einströmende zu dem Ausströmende des Rahmens (12) zu regulieren; und
Montieren einer Schürzenanordnung (15) an dem kranzförmigen Rahmen (12), wobei die Schürzenanordnung (15) ein Laminat mit einer Textilschicht (208; 300) umfasst, die sandwichartig zwischen einer ersten Verkapselungsschicht (206) und einer zweiten Verkapselungsschicht (204) angeordnet ist, wobei die erste und die zweite Verkapselungsschicht (204) aus einem Elastomer gefertigt sind, und die Textilschicht (208; 300) einen ersten Satz von Garnen (210; 302) und einen zweiten Satz von Garnen (212; 304), der mit dem ersten Satz von Garnen (210; 302) verwebt ist, umfasst, wobei der erste und der zweite Satz von Garnen nicht-rechtwinklig und nicht-parallel zu der längsgerichteten Achse (L) sind;
wobei die Textilschicht (208; 300) eine Webdichte von weniger als 150 ppi aufweist.

## Revendications

1. Valvule prothétique implantable (200 ; 300) comprenant :
un cadre annulaire (12) qui est radialement déployable à partir d'un état radialement comprimé vers un état radialement déployé, le cadre (12) présentant une extrémité d'entrée, une extrémité de sortie et un axe longitudinal (L) s'étendant à partir de l'extrémité d'entrée vers l'extrémité de sortie ;
une pluralité de feuillets (40) positionnés afin de réguler un écoulement de sang à partir de l'extrémité d'entrée vers l'extrémité de sortie du cadre (12) ; et
un ensemble collerette (15) comprenant un stratifié présentant une couche textile (208 ; 300) prise en sandwich entre une première couche d'encapsulation (206) et une seconde couche d'encapsulation (204), les première et seconde couches d'encapsulation (204) étant constituées d'un élastomère et la couche textile (208 ; 300) comprenant un premier ensemble de fils (210 ; 302) et un deuxième ensemble de fils (212 ; 304) entrelacés avec le premier ensemble de fils (210 ; 302), les premier et deuxième ensembles de fils étant non perpendiculaires et non parallèles à l'axe longitudinal (L) ;
la couche textile (208 ; 300) présentant une densité de tissage inférieure à 150 ppi, soit 150 duites par pouce, un pouce étant égal à 2,54 centimètres.

2. Valvule prothétique (200) selon la revendication 1, la couche textile (208 ; 300) présentant une densité de tissage de 50 ppi ou moins ; de préférence, la couche textile (208 ; 300) présentant une densité de tissage d'environ 30 à environ 50 ppi.

3. Valvule prothétique (200) selon l'une quelconque revendication précédente, l'ensemble collerette (15) comprenant une collerette externe (202) qui est montée sur une surface externe du cadre (12).

4. Valvule prothétique (200) selon l'une quelconque des revendications 1 à 3, l'ensemble collerette (15) comprenant une collerette interne (16) qui est montée sur une surface interne du cadre (12).

5. Valvule prothétique (200) selon l'une quelconque revendication précédente, la couche textile (208 ; 300) étant une couche tressée.

6. Valvule prothétique (200) selon l'une quelconque revendication précédente, les fils des premier et deuxième ensembles de fils étant orientés à des angles dans la plage de 20 à 70 degrés, dans la plage de 30 à 60 degrés, dans la plage de 40 à 50 degrés ou à des angles de 45 degrés par rapport à l'axe longitudinal (L) du cadre (12).

7. Valvule prothétique (200) selon l'une quelconque revendication précédente, les fils des premier et deuxième ensembles de fils présentant environ 10 à environ 50 filaments par fil ; de préférence les fils des premier et deuxième ensembles de fils présentant environ 20 filaments par fil.

8. Valvule prothétique (200) selon la revendication 7, les filaments des fils présentant une épaisseur située dans la plage d'environ 8 microns à environ 16 microns ; de préférence les filaments des fils présentant une épaisseur d'environ 10 microns.

9. Valvule prothétique (200) selon l'une quelconque revendication précédente, au moins certains des fils des premier et deuxième ensembles de fils étant texturés de telle sorte qu'ils peuvent conserver un état torsadé ou enroulé lorsque la valvule prothétique (200) est dans l'état radialement déployé et sont redressés vers un état non torsadé ou déroulé lorsque la valvule prothétique (200) est dans l'état radialement comprimé.

10. Valvule prothétique (200) selon l'une quelconque revendication précédente, la couche textile (208 ; 300) comprenant des fils tissés en armure gaze.

11. Valvule prothétique (200) selon l'une quelconque revendication précédente, les fils des premier et deuxième ensembles de fils comprenant un premier type de fils et un second type de fils, le premier type de fils étant non élastique ou moins élastique que le second type de fils.

12. Valvule prothétique (200) selon l'une quelconque revendication précédente, la couche textile (208 ; 300) comprenant un troisième ensemble de fils (308) s'étendant axialement et tressés conjointement avec les premier et deuxième ensembles de fils afin de former une tresse triaxiale.

13. Valvule prothétique (200) selon la revendication 12, le troisième ensemble de fils (308) étant constitués d'un élastomère qui est conçu pour s'étirer axialement lorsque la valvule prothétique (200) est comprimée radialement à partir de l'état radialement déployé vers l'état radialement comprimé ;
et/ou le troisième ensemble de fils (308) étant texturés de telle sorte qu'ils peuvent conserver un état torsadé ou enroulé lorsque la valvule prothétique (200) est dans l'état radialement déployé et sont redressés vers un état non torsadé ou déroulé lorsque la valvule prothétique (200) est dans l'état radialement comprimé.

14. Valvule prothétique (200) selon l'une quelconque revendication précédente, dans laquelle, lorsque la valvule prothétique (200) est dans l'état radialement comprimé, l'ensemble collerette (15) est allongé axialement jusqu'à au moins 40 % de sa longueur initiale lorsque la valvule prothétique (200) est dans l'état radialement déployé.

15. Procédé d'assemblage d'une valvule prothétique (200), comprenant :
le montage d'une pluralité de feuillets (40) sur un cadre annulaire (12) qui est radialement déployable à partir d'un état radialement comprimé vers un état radialement déployé, le cadre (12) présentant une extrémité d'entrée, une extrémité de sortie et un axe longitudinal (L) s'étendant à partir de l'extrémité d'entrée vers l'extrémité de sortie, la pluralité de feuillets (40) étant conçus pour réguler un écoulement de sang à partir de l'extrémité d'entrée vers l'extrémité de sortie du cadre (12) ; et
le montage d'un ensemble collerette (15) sur le cadre annulaire (12), l'ensemble collerette (15) comprenant un stratifié présentant une couche textile (208 ; 300) prise en sandwich entre une première couche d'encapsulation (206) et une seconde couche d'encapsulation (204), les première et seconde couches d'encapsulation (204) étant constituées d'un élastomère et la couche textile (208 ; 300) comprenant un premier ensemble de fils (210 ; 302) et un deuxième ensemble de fils (212 ; 304) entrelacés avec le premier ensemble de fils (210 ; 302), les premier et deuxième ensembles de fils étant non perpendiculaires et non parallèles à l'axe longitudinal (L) ;
la couche textile (208 ; 300) présentant une densité de tissage inférieure à 150 ppi.
